(19) Europäisches Patentamt
European Patent Office
Office européen des brevets

(11) Veröffentlichungsnummer: **0 152 860**
**B1**

(12) # EUROPÄISCHE PATENTSCHRIFT

(45) Veröffentlichungstag der Patentschrift: **14.06.89**

(51) Int. Cl.⁴: **A 61 K 31/545**

(21) Anmeldenummer: **85101220.3**

(22) Anmeldetag: **06.02.85**

(54) **Verwendung von Cephemverbindungen zur Herstellung eines Arzneimittels für die Modulation des Immunsystems.**

(30) Priorität: **17.02.84 DE 3405728**

(43) Veröffentlichungstag der Anmeldung: **28.08.85 Patentblatt 85/35**

(45) Bekanntmachung des Hinweises auf die Patenterteilung: **14.06.89 Patentblatt 89/24**

(84) Benannte Vertragsstaaten: **AT BE CH DE FR GB IT LI LU NL SE**

(56) Entgegenhaltungen:
**EP-A- 0 064 256**
**EP-A- 0 078 532**

**THE JOURNAL OF ANTIBIOTICS, Band 37, Nr. 12, December 1984, Seiten 1719-1726, Japan Antibiotics Research Association, Tokyo, JP; M. LIMBERT et al.: "Cefodizime, and aminothiazolyl cephalosporin. IV. Influence on the immune system"**

(73) Patentinhaber: **HOECHST AKTIENGESELLSCHAFT, Postfach 80 03 20, D-6230 Frankfurt am Main 80 (DE)**

(72) Erfinder: **Limbert, Michael, Dr., Am Alten Birnbaum 21, D-6238 Hofheim am Taunus (DE)**
Erfinder: **Dürckheimer, Walter, Dr., Im Lerchenfeld 45, D-6234 Hattersheim am Main (DE)**
Erfinder: **Schorlemmer, Hans-Ulrich,, Dr., Am Kirschenwald 2, D-3550 Marburg-Dagobertshausen (DE)**
Erfinder: **Dickneite, Gerhard, Dr., Zum Hieb 31, D-3550 Marburg (DE)**

**EP 0 152 860 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft die Verwendung von Cephemverbindungen der allgemeinen Formel I

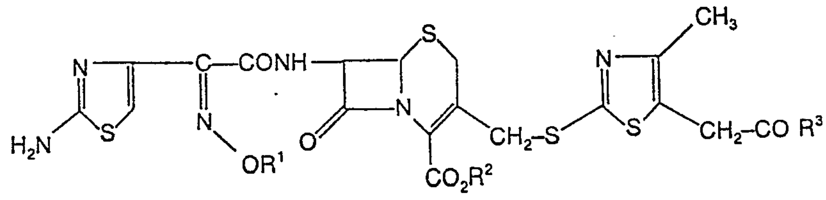

I

in der die Substituenten $R^1$, $R^2$ und $R^3$ die folgenden Bedeutungen besitzen

$R^1$ = $C_1$–$C_4$-Alkyl

$R^2$ = Wasserstoff

$R^3$ = Hydroxy, Phenyl-$C_1$–$C_2$-alkoxy, das durch Halogen oder $C_1$–$C_4$-Alkoxy substituiert sein kann, in der die $R^1$O-Gruppe in syn-Position steht und die Carboxylgruppen auch in Form ihrer physiologisch unbedenklichen Salze vorliegen können, zur Herstellung eines Arzneimittels für die Modulation des Immunsystems bei Mensch und Tier.

Die Substituenten $R^1$, $R^2$ und $R^3$ können beispielsweise die folgende Bedeutung besitzen.

Steht $R^1$ für Alkyl, so kommt ein solches mit 1 bis 4 C-Atomen in Betracht, vorzugsweise Methyl, Äthyl, n-Propyl, iso-Propyl, n-Butyl, iso-Butyl, besonders bevorzugt Methyl.

Als Kationen der physiologisch unbedenklichen Salze der Carboxylgruppen seien beispielsweise genannt Alkaliionen, insbesondere das Natrium- und Kaliumion, Erdalkaliionen, insbesondere das Calcium- und Magnesiumion, sowie ein Ammoniumion, vorzugsweise jedoch ein Natriumion, ein gegebenenfalls substituiertes alkyliertes Ammoniumion, wobei ein Alkylrest 1 bis 4 C-Atome haben kann, wie insbesondere Triäthylammonium, Diäthylammonium, Dimethylammonium oder Morpholinium, sowie auch basische Aminosäuren, wie z.B. Lysin oder Arginin in ihrer protonierten Form.

$R^3$ kann beispielsweise stehen für Hydroxy, für Phenylalkoxy, mit 1 bis 2 C-Atomen im Alkylteil, wobei der Phenylteil gegebenenfalls noch 1- oder 2-fach substituiert sein kann durch Halogen, vorzugsweise Chlor und Brom oder Alkoxy mit 1 bis 4 C-Atomen,
oder für Amino.

Im folgenden seien einige Gruppen aufgeführt, die erfindungsgemäß für $R^3$ von besonderem Interesse sind.

$R^3$ in der Bedeutung von Phenyl-$C_1$–$C_2$-alkoxy kann beispielsweise stehen für Benzyloxy, 4-Chlorbenzyloxy, 4-Methoxybenzyloxy, 3,4-Dimethoxybenzyloxy, und Phenäthoxy, bevorzugt jedoch für Benzyloxy, 4-Chlorbenzyloxy, 3,4-Dichlorbenzyloxy, 4-Methoxybenzyloxy, 3,4-Dimethoxybenzyloxy.

Für die erfindungsgemäße Verwendung besonders bevorzugt ist das Cefodizim (HR 221), das einer Verbindung der allgemeinen Formel I entspricht, in der $R_1$ = $CH_3$, $R_2$ = Na und $R_3$ = ONa bedeutet.

Verbindungen der allgemeinen Formel I und ihre Herstellung werden beispielsweise beschrieben in DE-OS 27 16 707, 31 17 438 und 31 43 537.

Es ist bekannt, daß der lebende Organismus über humorale und zelluläre immunologische Abwehrmechanismen verfügt. Sie dienen dazu, eingedrungene Fremdkörper, die pathogenetische Veränderungen hervorrufen können, zu neutralisieren und zu eliminieren. Es gibt zahlreiche Erkrankungen, die von einer Schwächung des Immunsystems begleitet sind, oder bei denen dessen Funktion nicht ausreicht, mit den Erregern, wie z.B. Mikroorganismen oder Krebszellen, fertigzuwerden. Man sucht deshalb seit langem nach immunmodulierenden Substanzen, die aufgrund ihrer hohen Wirksamkeit und guten Verträglichkeit einen breiten Einsatz zur Unterstützung der Abwehrkräfte des Körpers erlauben.

Die vorliegende Erfindung beschreibt eine neue Klasse von immunpharmakologisch wirksamen Substanzen, die chemisch definiert sind, geringe Toxizität besitzen und darüberhinaus hervorragende Antibiotika zur Bekämpfung lokaler und systemischer bakterieller Infektionen darstellen. Es ist bekannt, daß eine Reihe antibiotisch gut wirksamer Verbindungen wie z.B. Chloramphenicol oder Tetracyclin die Abwehrkräfte des Körpers negativ beeinflussen können. Es war deshalb überraschend, daß die erfindungsgemäßen Verbindungen in geringer Konzentration die Immunreaktion positiv beeinflussen, d.h. zur Steigerung der immunologischen Reaktivität führen, ohne toxische Nebenwirkungen zu zeigen.

Die neuen Verbindungen eignen sich damit nicht nur als Breitbandantibiotika, sondern entfalten eine zweite Hauptwirkung durch ihre immunstimulierenden Eigenschaften. Dies kann bei Patienten mit einer erworbenen oder angeborenen Abwehrschwäche von großer Bedeutung für den Heilungserfolg sein. Darüber hinaus können die neuen Verbindungen auch zum Schutz vor Infektionen oder zur beschleunigten Elimination von Mikroorganismen oder entarteter Zellen aus dem Körper eingesetzt werden. Im Tierversuch ist es beispielsweise möglich, eine tödlich verlaufende Candida-Infektion bei Mäusen durch prophylaktische Gabe von Cefodizim so günstig zu beeinflussen, daß am Ende des Beobachtungszeitraums

(14 Tage) eine Überlebensrate von > 65% resultiert. In der Kontrollgruppe überlebt im gleichen Beobachtungszeitraum kein Tier.

Die Wirkstoffe können erfindungsgemäß sowohl parenteral als auch oral verabreicht werden.

Die wirksame immunmodulatorische Menge liegt im Bereich von 1–200, vorzugsweise 10–50 mg pro kg Körpergewicht bei parenteraler Gabe. Der Wirkstoff kann allein verabreicht werden aber auch mit anderen Arzneimitteln, die Infektionen und Krebserkrankungen günstig beeinflussen, kombiniert werden. Für die orale und parenterale Gabe kommen Lösungen oder Suspensionen des Wirkstoffes in Betracht. Zur Herstellung wäßriger Lösungen wird der Wirkstoff vorzugsweise in Form wasserlöslicher, physiologisch verträglicher Salze wie sie sich beispielsweise aus den vorstehend angeführten Definitionen von $R^2$ ergeben, eingesetzt. Die Zubereitungen können die üblichen Hilfs- und Trägerstoffe enthalten. Als solche kommen beispielsweise Füllstoffe, Emulgatoren, Gleitstoffe und Pufferstoffe in Frage. Der Wirkstoff wird als freie Säure oder als Salz mit den galenischen Hilfsstoffen gemischt. Wird die freie Säure gewählt, so ist den Hilfsstoffen die äquivalente Menge einer entsprechenden Base zuzusetzen. Bei Verwendung des Wirkstoffes in Form von Suspensionen kommen als pharmazeutisch verträgliche Vektoren vorzugsweise hydroxylfreie Lösungsmittel, wie z.B. Pflanzenöle, in Frage.

Die folgenden Versuchsergebnisse sind Beispiele für die immunmodulierende Wirksamkeit der Verbindung der allgemeinen Formel I. Es wurden verschiedene Testmethoden herangezogen, die bekanntermaßen für die Beurteilung dieser Wirkqualität geeignet sind.

Beispiel 1

Stimulation von Maus-Peritonealmakrophagen

Makrophagen spielen bei der Abwehr von Infektionen bzw. bei der Immunreaktion eine zentrale Rolle. Einerseits wirken sie selbst bei der Elimination von Krankheitserregern mit, andererseits haben sie Kontrollfunktionen bei der Regulation der humoralen (B-Zell-abhängigen) und der zellulären (T-Zell-abhängigen) Immunreaktionen.

NMRI-Mäusen wurde Cefodizim 1 × intravenös in verschiedenen Konzentrationen zwischen 7,5 und 60 mg/kg Maus verabreicht. 72 h nach Gabe des Präparates wurden die Peritonealmakrophagen gewonnen und auf verschiedene Funktionen geprüft. Verglichen mit Makrophagen der Kontrollgruppe war die Chemilumineszenzreaktion der Makrophagen aus mit Cefodizim behandelten Tieren (15–60 mg/kg Maus) dosisabhängig signifikant erhöht (Tab. 1).

Die Makrophagen der mit Cefodizim (Formel I, $R^1 = CH_3$, $R^2 = Na$, $R^3 = ONa$) behandelten Mäuse zeichneten sich auch durch einen erhöhten Gehalt an lysosomalen Enzymen aus (Tab. 1).

Die Pinozytose von kolloidalem Gold (198 Au) durch Makrophagen aus mit Cefodizim behandelten Tieren war im Vergleich mit den Makrophagen aus unbehandelten Tieren signifikant erhöht (0,207 × $10^3$ cpm Kontrolle vs. 0,43 × $10^3$ der Makrophagen aus mit Cefodizim behandelten Tieren).

Tabelle 1

| Cefodizim Dosierung i.v. (mg/kg Maus) 1 × 72 h vor Test | Chemilumineszenzreaktion (RLU/15 min) | Gehalt an lysosomalen Enzymen (mU/ml) |
|---|---|---|
| 0 (Kontrolle) | $1,63 \times 10^5$ | $0,9 \times 10^3$ |
| 7,5 | $2,73 \times 10^5$ | $1,2 \times 10^3$ |
| 15 | $4,13 \times 10^5$ | $1,6 \times 10^3$ |
| 30 | $5,92 \times 10^5$ | $1,8 \times 10^3$ |
| 60 | $7,80 \times 10^5$ | $2,0 \times 10^3$ |

Beispiel 2

Verstärkung der immunologischen Reaktion vom verzögerten Typ (delayed type hypersensitivity, DTH)

Dieser Test gibt Auskunft über die Funktionsfähigkeit der T-Zell-abhängigen Komponente des Immunsystems. NMRI-Mäuse wurden mehrfach mit verschiedenen Dosen von Cefodizim intraperitoneal vorbehandelt. Am Tage der letzten Präparatgabe werden alle Tiere mit Schafbluterythrozyten i.v. immunisiert. Nach weiteren 5 Tagen wird die DTH-Reaktion auf eine intraplantare Injektion der Schafbluterythrozyten gemessen.

Es zeigt sich, daß bei mit Cefodizim vorbehandelten Tieren die DTH-Reaktion stärker ausfällt als bei entsprechenden Kontrolltieren (Tab. 2).

Tabelle 2

| Cefodizim Dosierung i.p. (mg/kg) über 4 Tage 2× täglich | DTH-Reaktion auf Schafbluterythrozyten Zunahme der Fußschwellung (%) |
|---|---|
| 0 (Kontrolle) | 21.6 |
| 30 | 26.9 |
| 40 | 30.5 |

Das Ergebnis dieses Versuchs zeigt, daß Cefodizim prophylaktisch verabreicht im T-Zell-System der Maus eine stimulierende Wirkung entfaltet.

Beispiel 3

Erhöhung der Widerstandskraft von Balb/c-Mäusen gegen eine Candida albicans-Infektion

Balb/c-Mäusen wird Cefodizim in einer Dosierung von 2 × 30 mg/kg/Tag über 4 Tage intraperitoneal verabreicht. 24 h nach der letzten Gabe von Cefodizim werden diese Tiere und die Kontrolltiere, denen physiologische Kochsalzlösung in gleichen Volumina und Zeitabständen verabreicht worden war, mit Candida albicans intravenös infiziert (5 × $10^6$ CFU/Maus). Die Tiere der Kontrollgruppe sterben durchschnittlich nach 3,5 Tagen, spätestens sterben alle Tiere nach 6 Tagen. Die

mit Cefodizim behandelte Gruppe überlebt zu > 65% nach einem Beobachtungszeitraum von 14 Tagen. Das Ergebnis diees Infektionsversuches spricht für eine Erhöhung der Infektresistenz der Balb/c-Mäuse gegen Candida albicans nach prophylaktischer Verabreichung von Cefodizim.

Beispiel 4
Stimulation der DTH-Reaktion und der Makrophagenaktivität durch verschiedene Derivate von Cefodizim

Wie bereits in den Beispielen 1 und 2 beschrieben, wurden NMRI-Mäuse parenteral mit verschiedenen Konzentrationen (10–200 mg/kg) Cefodizim und im Vergleich dazu mit Verbindungen der allgemeinen Formel I behandelt, in denen die Substituenten die folgenden Bedeutungen besitzen:

| Verbindung | $R_1$ | $R_2$ | $R_3$ |
|---|---|---|---|
| A | $-CH_3$ | H | 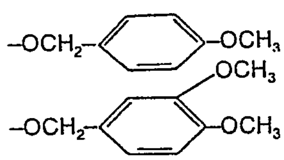 |
| B | $-CH_3$ | H | |
| C | $-CH_3$ | H | 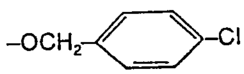 |
| D | $-CH_3$ | H | 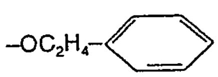 |
| E | $-CH_3$ | H | $-NH_2$ |

Als Tests zur Erfassung der Immunstimulation wurden die Funktion der Makrophagen (Chemilumineszenz und Enzymaktivität) und die DTH-Reaktion überprüft.

Die folgende Tabelle 3 zeigt die relative Wirksamkeit der einzelnen Substanzen, bezogen auf Cefodizim. Der 100%-Wert entspricht der maximalen Aktivierung (Differenz zwischen Kontrolle und Stimulation) durch Cefodizim, wie in Tabelle 1 und 2 angegeben.

Tabelle 3
Makrophagenaktivität

| Verbindung (10–200 mg/kg) | Chemilumineszenz | Exozytose | DTH-Reaktion (SRBC) |
|---|---|---|---|
| Cefodizim | 100% | 100% | 100% |
| A | 121% | 113% | 116% |
| B | 37% | 62% | 84% |
| C | 50% | 84% | 87% |
| D | 178% | 149% | 112% |
| E | 43% | 68% | 0% |

Der Tabelle ist zu entnehmen, daß im Vergleich zu Makrophagen aus unbehandelten Tieren, diese Zellen auch durch alle genannten Derivate, wie bereits für Cefodizim gezeigt, in ihrer Chemilumineszenzreaktion stark stimuliert und in ihrem Gehalt an lysosomalen Enzymen deutlich erhöht waren. Auch die DTH-Reaktion war bei den mit Cefodizim-Derivaten vorbehandelten Tieren deutlich stärker ausgeprägt, als bei den entsprechenden Kontrolltieren.

**Patentansprüche**

1. Verwendung von Cephemverbindung der allgemeinen Formel I

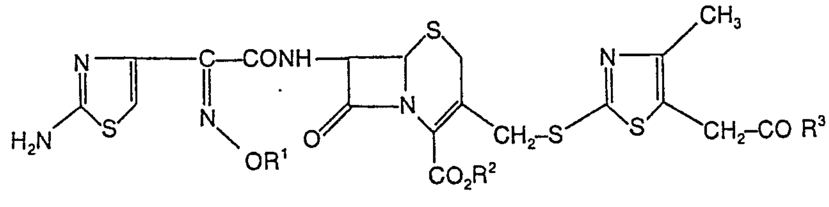

I

in der die Substituenten $R^1$, $R^2$ und $R^3$ die folgenden Bedeutungen besitzen
$R^1$ = $C_1$–$C_4$-Alkyl,
$R^2$ = Wasserstoff,
$R^3$ = Hydroxy, Phenyl-$C_1$–$C_2$-alkoxy, das durch Halogen oder $C_1$–$C_4$-Alkoxy substituiert sein kann, in der die $R^1$O-Gruppe in syn-Position steht und die Carboxylgruppen auch in Form ihrer physiologisch unbedenklichen Salze vorliegen können, zur Herstellung eines Arzneimittels für die Steigerung der immunologischen Reaktivität von Säugern.

2. Verwendung von Cephemverbindungen der allgemeinen Formel I

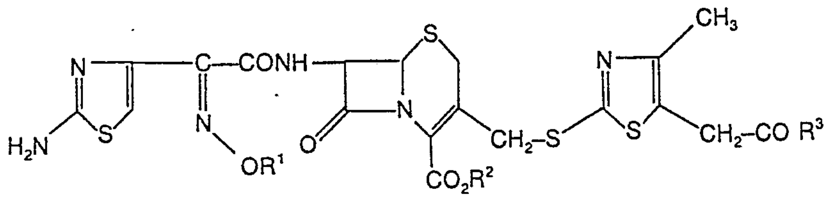

I

in der die Substituenten $R^1$, $R^2$ und $R^3$ die folgenden Bedeutungen besitzen

$R^1$ = Methyl,
$R^2$ = Na,
$R^3$ = ONa

und in der die $R^1$O-Gruppe in syn-Position steht, zur Herstellung eines Arzneimittels für die Steigerung der immunologischen Reaktivität von Säugern.

**Claims**

1. The use of cephem compounds of the general formula I

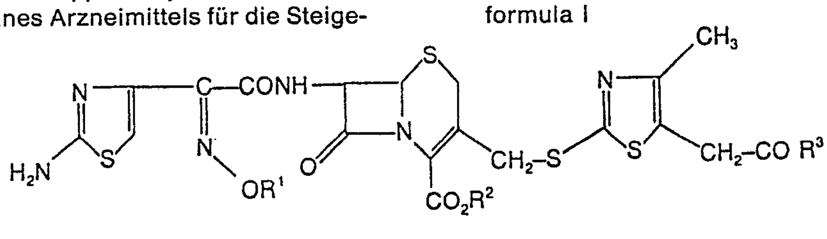

I

in which the substituents $R^1$, $R^2$ and $R^3$ have the following meanings

$R^1$ = $C_1$–$C_3$-alkyl,
$R^2$ = hydrogen,
$R^3$ = hydroxyl, phenyl-$C_1$–$C_2$-alkoxy which can be substituted by halogen or $C_1$–$C_4$-alkoxy,

in which the $R^1$O group is in the syn position, and the carboxyl groups can also be in the form of their physiologically acceptable salts, for the preparation of a pharmaceutical for increasing the immunological reactivity of mammals.

2. The use of cephem compounds of the general formula I

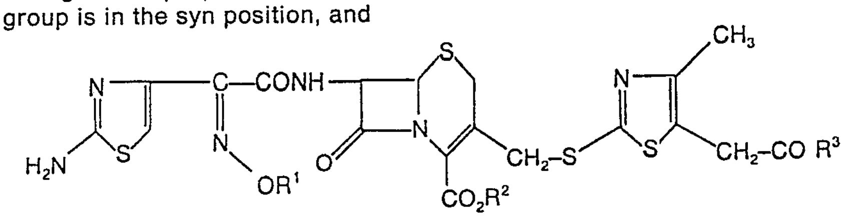

I

in which the substituents $R^1$, $R^2$ and $R^3$ have the following meanings

$R^1$ = methyl,
$R^2$ = Na,
$R^3$ = ONa

and in which the $R^1$O group is in the syn position, for the preparation of a pharmaceutical for increasing the immunological reactivity of mammals.

**Revendications**

1. Utilisation de dérivés de céphalosporine de formule générale I

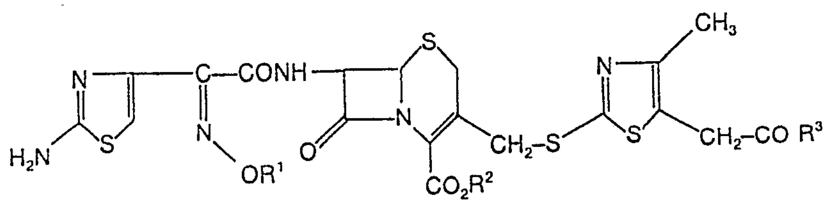

I

où $R^1$, $R^2$ et $R^3$ ont les significations suivantes

$R^1$ = alcoyle $C_1$–$C_4$
$R^2$ = hydrogène
$R^3$ = hydroxy, phényl-$C_1$–$C_2$-alcoxy, substitué ou non par un halogène ou un $C_1$–$C_4$-alcoxy,

dans lequel le groupe $R^1$O est en position syn et les groupes carboxyles peuvent aussi se présenter sous forme de leurs sels physiologiquement acceptables pour la fabrication d'un médicament augmentant l'immunoréactivité des mammifères.

2. Emploi de dérivés de céphalosporine de formule générale I

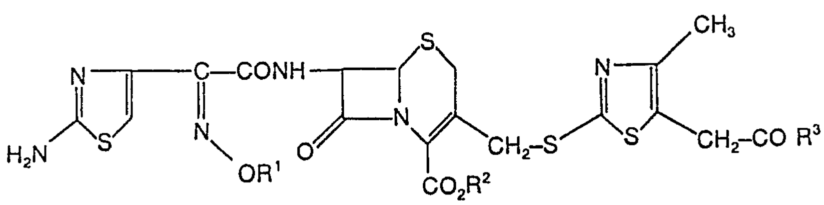

I

où $R^1$, $R^2$ et $R^3$ ont les significations suivantes

$R^1$ = méthyle,
$R^2$ = Na
$R^3$ = ONa

dans lequel le groupe $R^1$O est en position syn pour la fabrication d'un médicament augmentant l'immunoréactivité des mammifères.